# EUROPEAN PATENT SPECIFICATION

(11) **EP 2 938 282 B1**
(45) Date of publication and mention of the grant of the patent: **11.04.2018**
(21) Application number: 13824574.1
(22) Date of filing: 30.12.2013
(51) Int. Cl.: A61B 34/20, A61B 8/00

(54) **SYSTEMS OF REGISTRATION USING AN ULTRASOUND PROBE**
SYSTEME ZUR REGISTRIERUNG UNTER VERWENDUNG EINER ULTRASCHALLSONDE
SYSTÈMES DE RECALAGE AU MOYEN D'UNE SONDE ÉCHOGRAPHIQUE

(30) Priority: 31.12.2012 US 201261747784 P
(43) Date of publication of application: 04.11.2015
(73) Proprietor: Mako Surgical Corporation, Fort Lauderdale, FL 33317 (US)
(72) Inventor: KANG, Hyosig, Weston, Florida 33327 (US)
(74) Representative: Potter Clarkson LLP
(86) International application number: PCT/US2013/078221
(87) International publication number: WO 2014/106153

(56) References cited:
- EP-A1- 1 762 180
- WO-A1-2012/141184
- WO-A2-2006/092594
- US-A1- 2004 147 920
- US-A1- 2012 035 481

## Description

### CROSS-REFERENCE TO RELATED PATENT APPLICATIONS

This application claims the benefit of and priority to U.S. Provisional Application No. 61/747,784, filed December 31, 2012.

This application is related to U.S. Application No. 13/710,955, titled "Registration Using Phased Array Ultrasound," filed December 11, 2012.

### BACKGROUND

When performing computer-assisted surgery (CAS) and/or robotically-assisted surgery (RAS), it is often desirable to perform a registration step to align the computerized bone model with the actual patient bone. Existing systems achieve this goal by measuring point locations on the physical bone surface with a calibrated probe and feeding the point locations to the CAS/RAS system. Drawbacks of this approach include the need to use a sharp probe to penetrate the soft tissue surrounding the bone and the need to collect numerous intra-incision points. Document WO2012141184 A1 discloses an image processing apparatus comprising: shape obtaining means for obtaining information indicating a surface shape of a target object; discrimination means for discriminating a contact portion and a non-contact portion between the target object and an imaging surface of an ultrasonic probe which captures an ultrasonic image of the target object; position and orientation obtaining means for obtaining information indicating a position and orientation of the ultrasonic probe at the time of imaging; and alignment means for estimating deformation of the target object based on information indicating the surface shape, a discrimination result obtained by the discrimination means, and information indicating the position and orientation, and aligning the surface shape with the ultrasonic image.

### SUMMARY

One embodiment of the invention relates to a system for registration of a bone structure as defined in claim 1.

A method of registration of a bone structure includes receiving ultrasonic data of a patient's anatomy from a registration probe that includes a two-dimensional array of a plurality of ultrasonic transducers coupled to a rounded tip of the registration probe, wherein the ultrasonic transducers are configured to provide ultrasonic data of the patient's anatomy, determining which of the ultrasonic transducers are contacting the patient's anatomy, generating registration points based on the ultrasonic data of the patient's anatomy and the ultrasonic transducers that are contacting the patient's anatomy, and registering the patient's anatomy to a model of bone using the registration points.

Another embodiment of the invention relates to a non-transitory computer-readable medium having instructions stored thereon for execution by a processing circuit as defined in claim 11.

Alternative exemplary embodiments relate to other features and combinations of features as may be generally recited in the claims.

### BRIEF DESCRIPTION OF THE FIGURES

The disclosure will become more fully understood from the following detailed description, taken in conjunction with the accompanying figures, wherein like reference numerals refer to like elements, in which:
FIG. 1 is a diagram of a registration system in accordance with an exemplary embodiment.
FIG. 2 is a schematic diagram of a registration probe in accordance with an exemplary embodiment.
FIG. 3 is a schematic diagram of a registration probe tip including an ultrasound transducer array in accordance with an exemplary embodiment.
FIG. 4 is a schematic diagram of a registration probe tip including an ultrasound transducer array in accordance with an exemplary embodiment.
FIG. 5 is a flow diagram of a registration process in accordance with an exemplary embodiment.
FIG. 6 is a flow diagram of a registration process in accordance with an exemplary embodiment.
FIG. 7 is a flow diagram of a registration process in accordance with an exemplary embodiment.
FIG. 8 is a flow diagram of a registration process in accordance with an exemplary embodiment.

### DETAILED DESCRIPTION

Before turning to the figures, which illustrate the exemplary embodiments in detail, it should be understood that the application is not limited to the details or methodology set forth in the description or illustrated in the figures. It should also be understood that the terminology is for the purpose of description only and should not be regarded as limiting.

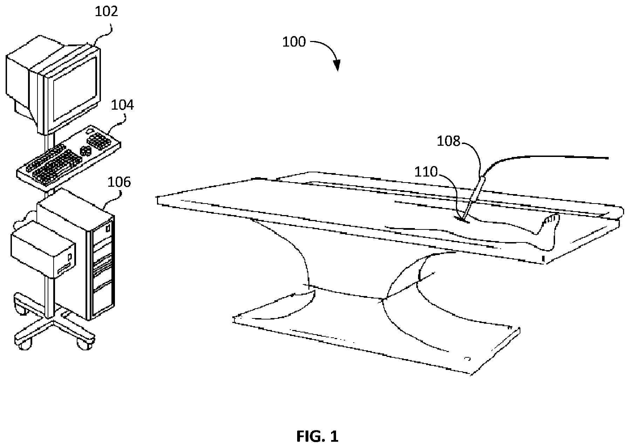

Referring to FIG. 1, a bone structure registration system 100 is shown according to an exemplary embodiment. Registration system 100 includes a processing circuit, depicted as computing device 106, and an ultrasound probe 108 having rounded tip. The rounded tip of the probe includes a two-dimensional array of ultrasonic transducers 110. Although any suitable ultrasound transducer technology, such as piezoelectric transducers, may be used in implementing the described systems and methods herein, in an exemplary embodiment, ultrasonic transducers 110 include capacitive micromachined ultrasound transducers (CMUTs). A CMUT includes two plate electrodes which are DC biased. One plate is driven with an additional AC signal. In addition to the substrate, the body of each CMUT includes a cavity, a membrane, and the electrode. Other layers may be included as needed, e.g., an insulating layer may also be included to prevent the two electrodes from coming into contact. A single transducer may be made up of multiple CMUTs in parallel, with the transducer elements then being arranged to form a desired array. An exemplary two-dimensional array of ultrasound transducers is described in detail in U.S. Application No. 13/710, 955. The ultrasonic transducers provide ultrasonic data relating to the anatomy of a patient to computing device 106, which processes the received ultrasonic data. Transmission of ultrasonic data from ultrasound probe 108 to computing device 106 may be implemented via wired or wireless communications according to any protocol(s) known to those of skill in the art. Computing device 106 generates registration points based on received ultrasonic data, determines which of the ultrasonic transducers are providing acceptable signals (e.g., which transducers are in contact with bone, cartilage, or other soft tissue), and registers the anatomy of the patient to a model of bone.

The processing circuit of bone structure registration system 100, which is represented in FIG. 1 as computing device 106, includes a processor and memory device. The processor can be implemented as a general purpose processor, an application specific integrated circuit (ASIC), one or more field programmable gate arrays (FPGAs), a group of processing components, or other suitable electronic processing components. The memory device (e.g., memory, memory unit, storage device, etc.) is one or more devices (e.g., RAM, ROM, Flash memory, hard disk storage, optical storage, etc.) for storing data and/or computer code for completing or facilitating the various processes and functions described in the present application. The memory device may be or include volatile memory or nonvolatile memory. The memory device may include database components, object code components, script components, or any other type of information structure for supporting the various activities and information structures described in the present application. According to an exemplary embodiment, the memory device is communicably connected to the processor via the processing circuit and includes computer code for executing (e.g., by the processing circuit and/or processor) one or more processes described herein.

In an exemplary embodiment, computing device 106 is configured to communicate with ultrasound probe 108. Furthermore, computing device 106 may receive information related to surgical procedures and may perform various functions related to performance of surgical procedures. For example, computing device 106 may have software modules as necessary to perform functions related to image analysis, surgical planning, registration, and navigation (e.g., of the probe or other surgical elements). As another example, computing device 106 may have software modules as necessary to control the operation of ultrasound probe 108. This includes the command set and protocol required to control the operation of the two-dimensional array of ultrasonic transducers 110 of ultrasound probe 108. The command set includes commands for enabling and disabling particular ultrasonic transducers of two-dimensional array of ultrasonic transducers 110, enabling and disabling subsets of two-dimensional array of ultrasonic transducers 110, and steering and focusing beams generated by the ultrasonic transducers.

In an exemplary embodiment, computing device 106 contains software for determining which specific ultrasonic transducers of two-dimensional array of ultrasonic transducers 110 are in contact (or are sufficiently close) to the anatomy of a patient (e.g., bone, cartilage, other soft tissue, etc.) to provide adequate ultrasonic signals. Computing device 106 may utilize filtering algorithms to filter signals from the two-dimensional array based on intensity. When the ultrasound probe 108 is contacting the anatomy of a patient, some portions of the two-dimensional array will contact the anatomy of the patient and other portions of the two-dimensional array will not. The portions that are not touching the anatomy of the patient will not be in direct acoustical contact with the patient. Without direct acoustical contact, an ultrasonic signal generated by an ultrasonic transducer will not result in a high quality return signal. An exemplary filtering algorithm may apply a threshold or high-pass filter to received signals in order to filter out lower intensity signals provided from transducers that are not in contact the patient's anatomy. In this manner, computing device 106 may determine which transducers are providing higher quality signals and are therefore contacting the patient's anatomy. Computing device 106 may utilize or otherwise prioritize this information in selecting a subset of transducers to receive data from, in generating registration points, and in registering the anatomy of the patient to a model of bone. It should be noted that the scope of the present application is not limited to a particular method of filtering. Additionally, an acoustic coupling medium may be disposed between the two-dimensional array of ultrasonic transducers 110 and a patient's skin to facilitate reliable transmission and receipt of ultrasound signals.

In an exemplary embodiment, computing device 106 contains software for registering the anatomy of the patient to a model of bone. Registration is based on the ultrasonic data received from ultrasound probe 108. In some embodiments, registration may also be supplemented by pressure data, in addition to location and tracking data. Computing device 106 may generate registration points using any received data from ultrasound probe 108 (e.g., by analyzing collected delay and amplitude data to identify a surface of the underlying bone structure). The registration points may correspond to certain locations or an identified surface of the patient to be mapped (or otherwise correlated or registered) to a model of bone. The registration point may then be mapped, e.g., via a best fit or otherwise, to a patient-specific model based on a prior imaging scan, such as a CT, MRI, or other scan type known to those of skill in the art. Alternatively, the registration points may be mapped to a model obtained utilizing an imageless system known to those of skill in the art, such as systems utilizing statistically shaped models and methods of bone morphing. In yet another embodiment, the registration points may be mapped to a general bone model.

A tracking system (such as an optical tracking system) can be used to track the location of the bone structure and the ultrasound probe 108. The tracking system may include trackable markers, such as optical arrays, which are fixed to the patient's bone structure, to the ultrasound probe 108, and to any other tracked objects within the registration system 100. Data related to the location of the tracked objects is received by computing device 106. The tracking data is used to determine the actual, physical location of the bone structure such that the physical location can be registered to the model of bone (which is in virtual, software space). Once the physical bone structure is registered to the virtual model of bone, the tracking system is able to accurately track the location of the bone structure during a surgical procedure.

In an exemplary registration process, computing device 106 determines the location and orientation of the two-dimensional array of ultrasonic transducers 110 of the ultrasound probe 108 (obtained by tracking a trackable marker fixed to the ultrasound probe 108) relative to the location and orientation of the trackable marker fixed to the bone structure. Computing device 106 causes each ultrasonic transducer to produce a signal and records the echo amplitude and delay of the signal. The echo amplitude and delay may be recorded in conjunction with a targeted location to produce a listing of associated locations and amplitudes/delays. Computing device 106 can then use the collected amplitude and delay data to register the underlying bone structure to a model of the structure.

FIG. 1 further includes display 102 and interface 104. Interface 104 may be a user interface that allows a clinician or other operator to interact with computing device 106 and ultrasound probe 108. Interface 104 may include a keyboard, a mouse, display 102, a touch screen, etc. Interface 104 allows a clinician to send and receive commands and control signals to ultrasound probe 108 for use as described herein. Computing device 106 may format data for use on display 102 (e.g., a CRT monitor, an LCD screen, etc.). For example, computing device 106 may generate appropriate signals such that received ultrasonic information is displayed as real time images.

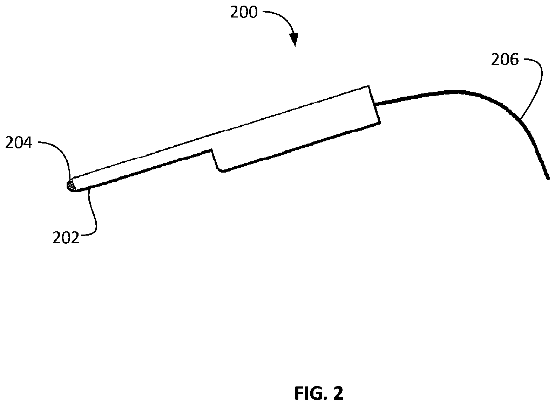

Referring to FIG. 2, a registration probe 200 is shown in accordance with an exemplary embodiment. Registration probe 200 may be configured for use internally (e.g., through an incision) or externally (e.g., on a patient's skin). Registration probe 200 includes a rounded (or otherwise curved) tip 202, which may be rigid or flexible in nature. Rounded tip 202 includes an array of ultrasonic transducers 204. The array of ultrasonic transducers 204 may be a two-dimensional array of ultrasonic transducers 110 as described in connection with FIG. 1. The array of ultrasonic transducers 204 is operably coupled to rounded tip 202. The array of ultrasonic transducers 204 may be coupled to rounded tip 202 using any manner known to those of skill in the art. In some embodiments, array of ultrasonic transducers 204 may also be removable, or otherwise replaceable. For example, a particular array of ultrasonic transducers 204 with certain dimensions or sensor characteristics may be selected for a particular purpose. In an exemplary embodiment, the array of ultrasonic transducers 204 is formed from an array of capacitive micromachined ultrasonic transducer (CMUT) devices.

In another embodiment, the array of ultrasonic transducers 204 may also include pressure sensing devices. Exemplary pressure sensing devices include microelectromechanical systems (MEMS) pressure sensors, although other pressure sensing devices are envisioned. Such pressure sensing devices are configured to provide pressure data (e.g., force data) to a computing device (e.g., computing device 106 of FIG. 1). Pressure data is used by the computing device to supplement the determination of which transducers are in contact with a patient's anatomy. As an example, the magnitude of a pressure signal provided may be evaluated to determine which transducers are in contact with the patient. The magnitude may be evaluated in light of values representing the rigidity of bone, cartilage, or other patient contact surface. In one embodiment, pressure sensing devices are interspersed between ultrasonic transducers. In another embodiment, pressure sensing devices may be part of a separate array coupled to array of ultrasonic transducers 204 (e.g., underneath the array of ultrasonic transducers 204).

In the exemplary embodiment shown in FIG. 2, the registration probe further includes communication interface 206. Communication interface 206 may be a cord or wireless transmission mechanism for connecting registration probe 200 to a computing device. Communication interface 206 facilitates the transmission and reception of data between registration probe 200 and the computing device. Data may include control signals, commands, ultrasonic information, pressure data, etc.

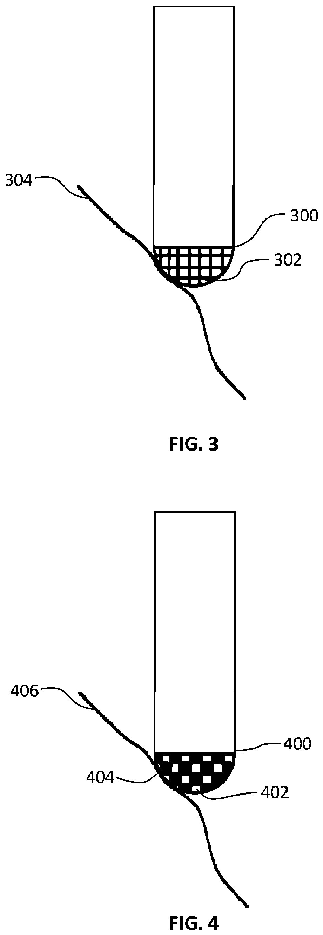

Referring to FIG. 3, a registration probe tip 300 is shown in accordance with an exemplary embodiment. The registration probe tip 300 includes an array of ultrasonic transducers 302. The array of ultrasonic transducers 302 may be an array of ultrasonic transducers as described herein (e.g., a two-dimensional array of ultrasonic transducers 110 of FIG. 1). Registration probe tip 300 is depicted as curved in nature, and may be flexible or rigid. The array of ultrasonic transducers 302 is operably coupled to registration probe tip 300. In an exemplary embodiment, the array of ultrasonic transducers 302 includes capacitive micromachined ultrasonic transducer (CMUT) devices configured in a two-dimensional array. Each of the CMUT devices provides ultrasonic signals (e.g., source signals, echo, and reflection information) that may be used by a computing device in registering the anatomy of the patient to a model of bone. Each of the CMUT devices may be controlled by a computing device individually, in subsets, or as entire array. Such an array of CMUT devices is capable of sending and receiving signals at high frequencies, which is useful in allowing a clinician to quickly gather a high number of registration points. A computing device may process received ultrasonic information as described herein to determine which of the CMUT devices are in contact with the anatomy of a patient. Based on the amplitude and corresponding delay of ultrasonic reflections detected by the CMUT devices, the computing device may further determine the nature of the patient anatomy being scanned (e.g., bone, cartilage, other soft tissue, etc.). In another embodiment, piezoelectric transducers may be used in implementing the described systems and methods herein.

FIG. 3 also depicts patient anatomy 304 in contact with a portion of registration probe tip 300. Patient anatomy 304 is shown as curved in nature, but may also be flat, and may relate to any anatomy being scanned. Patient anatomy 304 may include bone, cartilage, or other soft tissue. Due to the curved nature of registration probe tip 300, at a point of contact (such as that shown with patient anatomy 304), there will be ultrasonic transducers that are in proper contact with the patient anatomy 304 for imaging purposes. The curved probe tip in combination with the two-dimensional array of transducers thus ensures the sufficient transducer contact to adequately image the bone surface. By implementing the contact detection systems discussed herein, a computing device may eliminate data from or disable ultrasonic transducers that are not in proper contact with the anatomy (e.g., ultrasonic transducers with signals indicating attenuation or loss). The computing device may continue to receive data from ultrasonic transducers indicating strong signals with minimal attenuation. In this manner, the computing device may obtain the points required to perform registration with an improved accuracy and precision. Such enabling and disabling of ultrasonic transducers may be controlled by a computing device in real time. In another embodiment, a computing device may receive data from all transducers, but may accept or ignore data from specific ultrasonic transducers in real time to achieve the same effect as enabling or disabling transducers.

Referring to FIG. 4, registration probe tip 400 is shown in accordance with an exemplary embodiment. Registration probe tip 400 includes an array of ultrasonic transducers 402. The array of ultrasonic transducers 402 is similar to the other arrays described herein (e.g., the two-dimensional array of ultrasonic transducers 110 of FIG. 1), but includes pressure sensors 404 interspersed among the ultrasonic transducers 402. Pressure sensors 404 are configured to provide pressure data corresponding to forces generated via contact with a patient's anatomy. Each of the pressure sensors may provide data to a computing device individually, in subsets, or as entire array. Pressure data is transmitted to the computing device (e.g., computing device 106 of FIG. 1) and evaluated to determine which ultrasonic transducers, or which portions of registration probe tip, are in contact with the anatomy of a patient. Contact detection using pressure data may supplement or replace contact detection processes that utilize filtering techniques as described above. In one embodiment, registration probe tip 400 includes a pressure sensor for each ultrasonic transducer. In another embodiment, registration probe tip 400 includes pressure sensors configured to provide force data corresponding to a subset of ultrasonic transducers. Pressure sensors may include such devices as pressure transducers, pressure transmitters, pressure senders, pressure indicators and piezometers, manometers, or micro electromechanical systems (MEMS).

In an exemplary embodiment, pressure sensors 404 are MEMS devices. Such MEMS devices are typically constructed using layers of polysilicon and layers of metals, and may be configured as pressure sensors as known to those of skill in the art. A MEMS pressure sensor may provide data relating to capacitance changes, which result from contact of a mechanical structure with a patient's anatomy. The data from the MEMS pressure sensor may be further processed to determine pressure values. As depicted in FIG. 4, pressure data provided by pressure sensors 404 may be used by a computing device in determining contact points on patient anatomy 406. Although pressure sensors 404 are shown as interspersed between the array of ultrasonic transducers 402, other embodiments may include other configurations, such as placing pressure sensors behind the array of ultrasonic transducers.

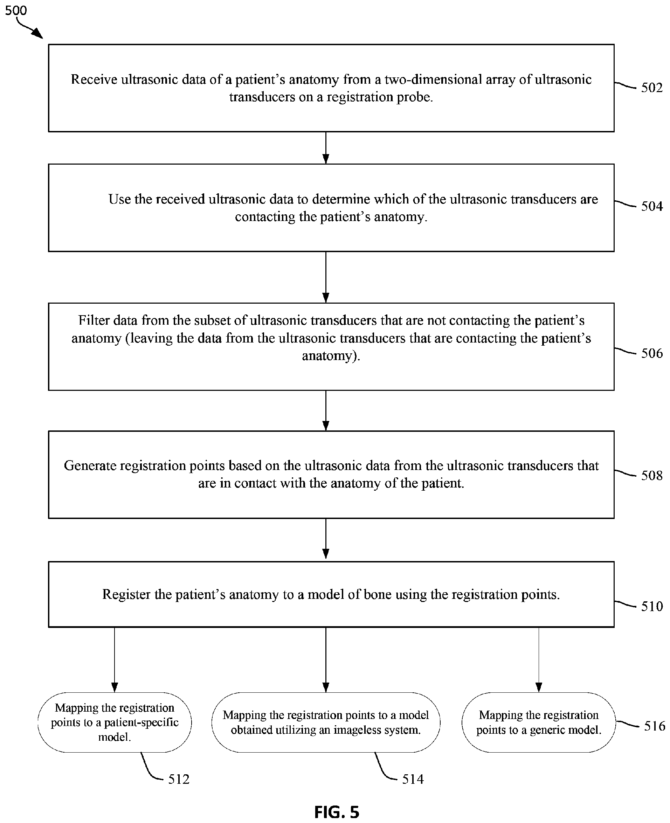

Referring to FIG. 5, a flow diagram of a bone structure registration process 500 is shown in accordance with an exemplary embodiment. In step 502, ultrasonic data corresponding to a patient's anatomy is received from a two-dimensional array of ultrasonic transducers on a registration probe, as described herein. In step 504, the received ultrasonic data is processed to determine which of the ultrasonic transducers are contacting the patient's anatomy. In step 506, the data is filtered to remove the data from the subset of ultrasonic transducers that are not contacting the patient's anatomy. In step 508, registration points are generated based on the ultrasonic data from the ultrasonic transducers in contact with the anatomy of the patient. In step 510, the patient's anatomy is registered to a model of bone using the generated registration points. Registration may be implemented using a variety of methods, including mapping the registration points to a patient-specific model (step 512), mapping the registration points to model obtained by utilizing an imageless system (step 514), or mapping the registration points to a generic model (step 516). Other methods of registration, as known to those skilled in the art, are also envisioned.

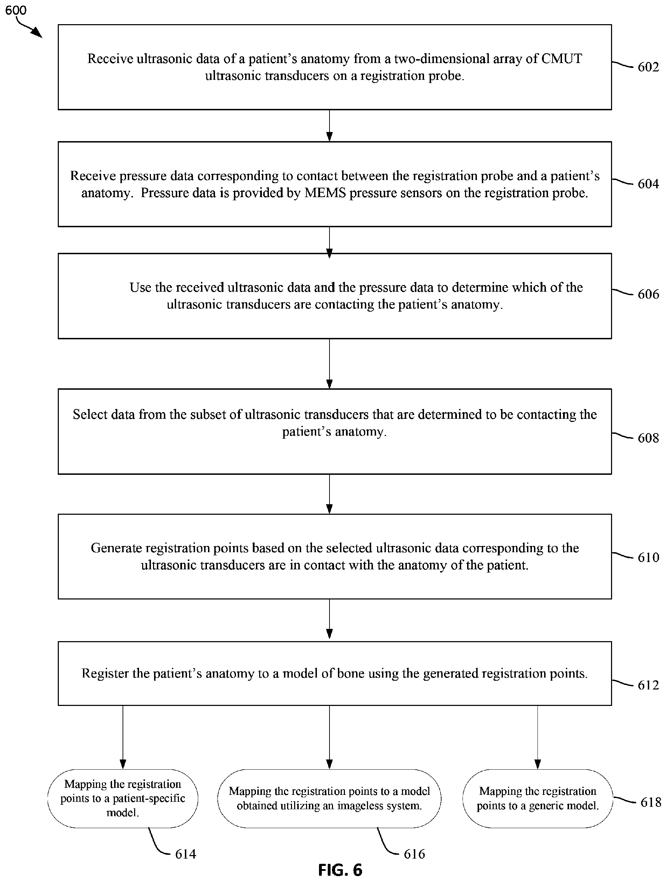

Referring to FIG. 6, a flow diagram of a bone structure registration process 600 is shown in accordance with an exemplary embodiment. In step 602, ultrasonic data corresponding to a patient's anatomy is received from a two-dimensional array of CMUT ultrasonic transducers on a registration probe, as described herein. In step 604, pressure data is received corresponding to contact between the registration probe and a patient's anatomy. The pressure data is provided by MEMS pressure sensors on the registration probe. In step 606, the received ultrasonic data and the pressure data are used to determine which of the ultrasonic transducers are contacting the patient's anatomy. This determination may make use of filtering techniques as discussed herein. In step 608, data is selected from the subset of ultrasonic transducers that are determined to be contacting the patient's anatomy. In step 610, registration points are generated based on the selected ultrasonic data corresponding to the ultrasonic transducers that are in contact with the anatomy of the patient. In step 612, the patient's anatomy is registered to a model of bone using the generated registration points. Registration may be implemented using a variety of methods, including mapping the registration points to a patient-specific model (step 614), mapping the registration points to model obtained by using an imageless system (step 616), or mapping the registration points to a generic model (step 618). Other methods of registration, as known to those skilled in the art, are also envisioned.

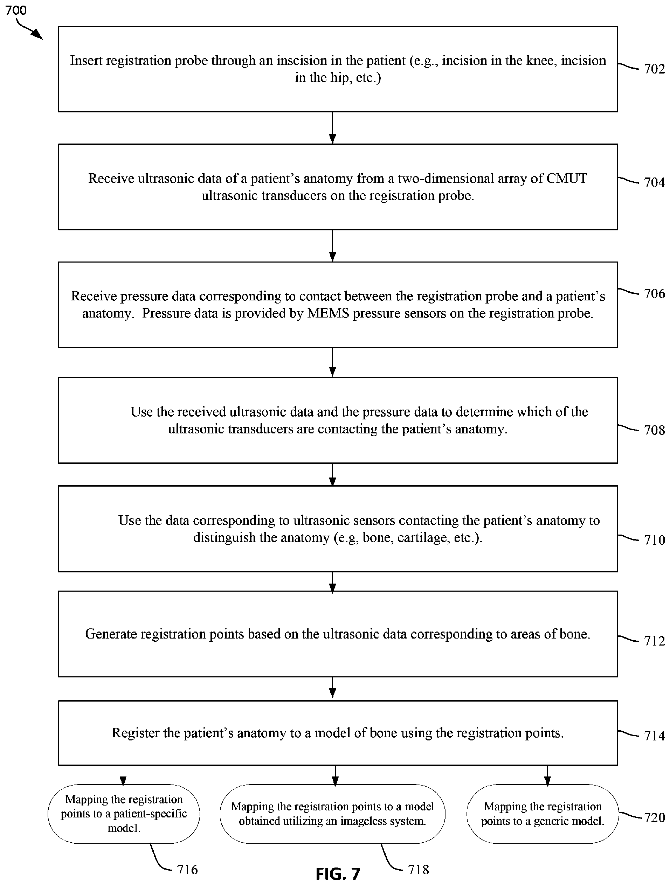

Referring to FIG. 7, a flow diagram of a bone structure registration process 700 is shown in accordance with an exemplary embodiment. In step 702, a registration probe as described herein is inserted through an incision in the patient (e.g., incision in the knee, incision in the hip, etc.). In step 704, ultrasonic data corresponding to a patient's anatomy is received from the two-dimensional array of CMUT ultrasonic transducers on the registration probe. In step 706, pressure data is received corresponding to contact between the registration probe and a patient's anatomy. The pressure data is provided by MEMS pressure sensors on the registration probe. In step 708, the received ultrasonic data and the pressure data are used (e.g., by a computing device) to determine which of the ultrasonic transducers are contacting the patient's anatomy. In step 710, the data corresponding to ultrasonic sensors contacting the patient's anatomy is further processed to distinguish the anatomy (e.g., bone, cartilage, etc.). In step 712, registration points are generated based on the ultrasonic data corresponding to areas of bone. In step 714, the patient's anatomy is registered to a model of bone using the generated registration points. Registration may be implemented using a variety of methods, including mapping the registration points to a patient-specific model (step 716), mapping the registration points to model obtained by using an imageless system (step 718), or mapping the registration points to a generic model (step 720). Other methods of registration, as known to those skilled in the art, are also envisioned.

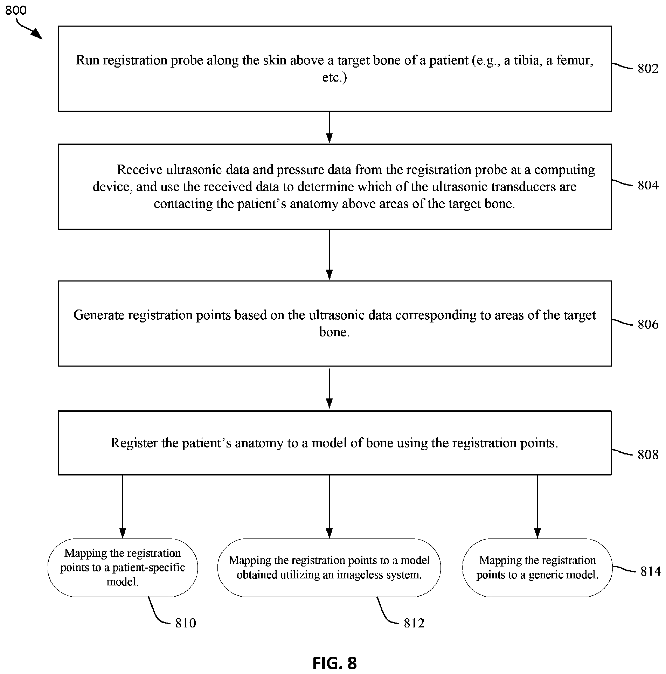

Referring to FIG. 8, a flow diagram of a bone structure registration process 800 is shown in accordance with an exemplary embodiment. In step 802, a registration probe (e.g., ultrasound probe 108 of FIG. 1) is guided along the skin above a target bone of a patient (e.g., a tibia, a femur, etc.). In step 804, ultrasonic data and pressure data are received from the registration probe by a computing device. The computing device uses the received data to determine which of the ultrasonic transducers are contacting the patient's anatomy above areas of the target bone. In step 806, registration points are generated based on the ultrasonic data corresponding to areas of the target bone. In step 808, the patient's anatomy is registered to a model of bone using the generated registration points. Registration may be implemented using a variety of methods, including mapping the registration points to a patient-specific model (step 810), mapping the registration points to model obtained by using an imageless system (step 812), or mapping the registration points to a generic model (step 814). Other methods of registration, as known to those skilled in the art, are also envisioned.

The present disclosure contemplates methods, systems and program products on any machine-readable media for accomplishing various operations. The embodiments of the present disclosure may be implemented using existing computer processors, or by a special purpose computer processor for an appropriate system, incorporated for this or another purpose, or by a hardwired system. Embodiments within the scope of the present disclosure include program products comprising machine-readable media for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage, other magnetic storage devices, solid state storage devices, or any other medium which can be used to carry or store desired program code in the form of machine-executable instructions or data structures and which can be accessed by a general purpose or special purpose computer or other machine with a processor. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general purpose computer, special purpose computer, or special purpose processing machines to perform a certain function or group of functions.

Although a specific order of method steps may be described, the order of the steps may differ from what is described. Fewer, additional, and/or different operations may be performed. Also, two or more steps may be performed concurrently or with partial concurrence. Such variation will depend on the software and hardware systems chosen and on designer choice. All such variations are within the scope of the disclosure. Likewise, software implementations could be accomplished with standard programming techniques with rule based logic and other logic to accomplish any connection steps, processing steps, comparison steps, and decision steps.

## Claims

1. A system (100) for registration of a bone structure, comprising:
a registration probe (108; 200) having a two-dimensional array of a plurality of ultrasonic transducers (110; 204; 302; 402) coupled to a rounded tip (202; 300; 400) of the probe (108; 200), wherein the ultrasonic transducers (110; 204; 302; 402) are configured to provide ultrasonic data; and
a processing circuit (106) configured to:
receive ultrasonic data from the plurality of ultrasonic transducers (110; 204; 302; 402); determine which of the ultrasonic transducers (110; 204; 302; 402) are in contact with anatomy (304; 406) of a patient; register the anatomy (304; 406) of the patient to a model of bone using registration points; the system being **characterised in that** the processing circuit is further configured to:
select a subset of the ultrasonic transducers (110; 204; 302; 402) based on the determination of which of the ultrasonic transducers (110; 204, 302, 402) are in contact with anatomy (304; 406) of the patient, the ultrasonic transducers of the subset being the ultrasonic transducers that are in contact with anatomy of the patient; generate the registration points based on the ultrasonic data of the selected subset of ultrasonic transducers (110; 204; 302; 402).

2. The system (100) of claim 1, wherein the ultrasonic transducers (110; 204; 302; 402) include capacitive micromachined ultrasonic transducer (CMUT) devices.

3. The system (100) of claim 1, wherein determining which of the ultrasonic transducers (110; 204; 302; 402) are in contact with the anatomy (304; 406) of the patient includes filtering based on an intensity of an ultrasonic signal.

4. The system (100) of claim 1, wherein the registration probe (108; 200) further comprises a plurality of pressure sensors (404) coupled to the rounded tip (400) of the probe (108; 200).

5. The system (100) of claim 4, wherein the pressure sensors (404) are interspersed between the ultrasonic transducers (402).

6. The system (100) of claim 4, wherein the pressure sensors (404) include microelectromechanical systems pressure sensors.

7. The system (100) of claim 4, wherein the pressure sensors (404) are configured to provide pressure data, and wherein the processing circuit (106) is further configured to receive pressure data from the pressure sensors (404).

8. The system (100) of claim 7, wherein determining which of the ultrasonic transducers (402) are in contact with the anatomy (406) of the patient is further based on the pressure data.

9. The system (100) of claim 1, wherein the model of bone is based on at least one of a CT scan or an MRI.

10. The system (100) of claim 1, wherein the model of bone is a model obtained utilizing an imageless system.

11. A non-transitory computer-readable medium having instructions stored thereon for execution by a processing circuit (106), the instructions comprising:
instructions to receive ultrasonic data of a patient's anatomy (304; 406) from a registration probe that includes an array of a plurality of ultrasonic transducers (110; 204; 302; 402) coupled to a rounded tip of a probe, wherein the ultrasonic transducers (110; 204; 302; 402) are configured to provide ultrasonic data of the patient's anatomy (304; 406);
instructions to determine which of the ultrasonic transducers (110; 204; 302; 402) are contacting the patient's anatomy (304; 406); instructions to register the patient's anatomy (304; 406) to a model of bone using registration points; **characterised in that** the instructions further comprise:
instructions to select a subset of the plurality of ultrasonic transducers (110; 204; 302; 402) based on the determination of which of the ultrasonic transducers (110; 204, 302, 402) are in contact with the patient's anatomy (304; 406), the ultrasonic transducers of the subset being the ultrasonic transducers that are in contact with anatomy of the patient; instructions to generate the registration points based on the ultrasonic data of the selected subset of ultrasonic transducers (110; 204; 302; 402).

12. The non-transitory computer-readable medium of claim 11, further comprising instructions to receive pressure data from pressure sensors (404), wherein the registration probe (108; 200) further includes a plurality of pressure sensors (404) coupled to the rounded tip (400) of the probe (108; 200), wherein the pressure sensors (404) are configured to provide pressure data, and wherein determining which of the ultrasonic transducers (402) are contacting the patient's anatomy (406) is further based on the pressure data.

## Patentansprüche

1. System (100) zum Registrieren einer Knochenstruktur, Folgendes umfassend:
eine Registrierungssonde (108; 200), aufweisend eine zweidimensionale Anordnung mehrerer Ultraschallwandler (110; 204; 302; 402), die mit einer abgerundeten Spitze (202; 300; 400) der Sonde (108; 200) gekoppelt sind, wobei die Ultraschallwandler (110; 204; 302; 402) dazu ausgelegt sind, Ultraschalldaten bereitzustellen; und
einen Verarbeitungsschaltkreis (106), zu Folgendem ausgelegt:
Empfangen von Ultraschalldaten von den mehreren Ultraschallwandlern (110; 204; 302; 402);
Bestimmen, welche der Ultraschallwandler (110; 204; 302; 402) sich in Berührung mit Anatomie (304; 406) eines Patienten befinden; Registrieren der Anatomie (304; 406) des Patienten an einem Knochenmodell unter Verwendung von Registrierungspunkten; wobei das System **dadurch gekennzeichnet ist, dass** der Verarbeitungsschaltkreis ferner zu Folgendem ausgelegt ist: Wählen eines Teilsatzes aus den Ultraschallwandlern (110; 204; 302; 402) basierend auf der Bestimmung, welche der Ultraschallwandler (110; 204, 302, 402) sich in Berührung mit Anatomie (304; 406) des Patienten befinden, wobei die Ultraschallwandler aus dem Teilsatz die Ultraschallwandler sind, die sich in Berührung mit Anatomie des Patienten befinden;
Generieren der Registrierungspunkte basierend auf den Ultraschalldaten des ausgewählten Teilsatzes von Ultraschallwandlern (110; 204; 302; 402).

2. System (100) nach Anspruch 1, wobei die Ultraschallwandler (110; 204; 302; 402) kapazitive mikromechanische Ultraschallwandler (capacitive micromachined ultrasonic transducers - CMUT) beinhalten.

3. System (100) nach Anspruch 1, wobei Bestimmen, welche der Ultraschallwandler (110; 204; 302; 402) sich in Berührung mit der Anatomie (304; 406) des Patienten befinden, Filtern basierend auf einer Intensität eines Ultraschallsignals beinhaltet.

4. System (100) nach Anspruch 1, wobei die Registrierungssonde (108; 200) ferner mehrere Drucksensoren (404), die mit der abgerundeten Spitze (400) der Sonde (108; 200) gekoppelt sind, umfasst.

5. System (100) nach Anspruch 4, wobei die Drucksensoren (404) zwischen den Ultraschallwandlern (402) verteilt sind.

6. System (100) nach Anspruch 4, wobei die Drucksensoren (404) Mikroelektromechanische-Systeme-Drucksensoren beinhalten.

7. System (100) nach Anspruch 4, wobei die Drucksensoren (404) dazu ausgelegt sind, Druckdaten bereitzustellen, und wobei der Verarbeitungsschaltkreis (106) ferner dazu ausgelegt ist, Druckdaten von den Drucksensoren (404) zu empfangen.

8. System (100) nach Anspruch 7, wobei Bestimmen, welche der Ultraschallwandler (402) sich in Berührung mit der Anatomie (406) des Patienten befinden, ferner auf den Druckdaten basiert.

9. System (100) nach Anspruch 1, wobei das Knochenmodell auf einer Computertomographieaufnahme und/oder einer MRT basiert.

10. System (100) nach Anspruch 1, wobei das Knochenmodell ein Modell ist, das unter Verwendung eines bildlosen Systems erhalten wird.

11. Nichtflüchtiges rechnerlesbares Medium, aufweisend darauf gespeicherte Befehle zum Ausführen durch einen Verarbeitungsschaltkreis (106), wobei die Befehle Folgendes umfassen: Befehle zum Empfangen von Ultraschalldaten einer Anatomie (304; 406) eines Patienten von einer Registrierungssonde, die eine Anordnung von mehreren Ultraschallwandlern (110; 204; 302; 402) beinhaltet, die mit einer abgerundeten Spitze einer Sonde gekoppelt sind, wobei die Ultraschallwandler (110; 204; 302; 402) dazu ausgelegt sind, Ultraschalldaten der Anatomie (304; 406) des Patienten bereitzustellen;
Befehle zum Bestimmen, welche der Ultraschallwandler (110; 204; 302; 402) die Anatomie (304; 406) des Patienten berühren;
Befehle zum Registrieren der Anatomie (304; 406) des Patienten an einem Knochenmodell unter Verwendung von Registrierungspunkten; **dadurch gekennzeichnet, dass** die Befehle ferner Folgendes umfassen:
Befehle zum Auswählen eines Teilsatzes aus den mehreren Ultraschallwandlern (110; 204; 302; 402) basierend auf der Bestimmung dessen, welche der Ultraschallwandler (110; 204, 302, 402) sich in Berührung mit der Anatomie (304; 406) des Patienten befinden, wobei die Ultraschallwandler des Teilsatzes die Ultraschallwandler sind, die sich in Berührung mit Anatomie des Patienten befinden;
Befehle zum Generieren der Registrierungspunkte basierend auf den Ultraschalldaten des ausgewählten Teilsatzes von Ultraschallwandlern (110; 204; 302; 402).

12. Nichtflüchtiges rechnerlesbares Medium nach Anspruch 11, ferner umfassend Befehle zum Empfangen von Druckdaten von Drucksensoren (404), wobei die Registrierungssonde (108; 200) mehrere Drucksensoren (404) beinhaltet, die mit der abgerundeten Spitze (400) der Sonde (108; 200) gekoppelt sind, wobei die Drucksensoren (404) dazu ausgelegt sind, Druckdaten bereitzustellen, und wobei Bestimmen, welche der Ultraschallwandler (402) die Anatomie (406) des Patienten berühren, ferner auf den Druckdaten basiert.

## Revendications

1. Système (100) d'alignement d'une structure d'os, comprenant :
une sonde d'alignement (108 ; 200) ayant un réseau bidimensionnel d'une pluralité de transducteurs ultrasoniques (110 ; 204 ; 302 ; 402) couplés à un bout arrondi (202 ; 300 ; 400) de la sonde (108 ; 200), dans lequel les transducteurs ultrasoniques (110 ; 204 ; 302 ; 402) sont configurés pour fournir des données ultrasoniques ; et
un circuit de traitement (106) configuré pour :
recevoir des données ultrasoniques provenant de la pluralité de transducteurs ultrasoniques (110 ; 204 ; 302 ; 402) ; déterminer lesquels des transducteurs ultrasoniques (110 ; 204 ; 302 ; 402) sont en contact avec l'anatomie (304 ; 406) d'un patient ; aligner l'anatomie (304 ; 406) du patient avec un modèle d'os à l'aide de points d'alignement ; le système étant **caractérisé en ce que** le circuit de traitement est en outre configuré pour : sélectionner un sous-ensemble des transducteurs ultrasoniques (110 ; 204 ; 302 ; 402) d'après la détermination des transducteurs ultrasoniques (110 ; 204 ; 302 ; 402) qui sont en contact avec l'anatomie (304 ; 406) du patient, les transducteurs ultrasoniques du sous-ensemble étant les transducteurs ultrasoniques qui sont en contact avec l'anatomie du patient ;
générer les points d'alignement d'après les données ultrasoniques du sous-ensemble sélectionné de transducteurs ultrasoniques (110 ; 204 ; 302 ; 402).

2. Système (100) selon la revendication 1, dans lequel les transducteurs ultrasoniques (110 ; 204 ; 302 ; 402) comportent des dispositifs de transducteurs ultrasoniques micro-usinés capacitifs (CMUT).

3. Système (100) selon la revendication 1, dans lequel la détermination des transducteurs ultrasoniques (110 ; 204 ; 302 ; 402) qui sont en contact avec l'anatomie (304 ; 406) du patient comporte un filtrage d'après une intensité d'un signal ultrasonique.

4. Système (100) selon la revendication 1, dans lequel la sonde d'alignement (108 ; 200) comprend en outre une pluralité de capteurs de pression (404) couplés au bout arrondi (400) de la sonde (108 ; 200).

5. Système (100) selon la revendication 4, dans lequel les capteurs de pression (404) sont disséminés entre les transducteurs ultrasoniques (402).

6. Système (100) selon la revendication 4, dans lequel les capteurs de pression (404) comportent des capteurs de pression de microsystèmes électromécaniques.

7. Système (100) selon la revendication 4, dans lequel les capteurs de pression (404) sont configurés pour fournir des données de pression, et dans lequel le circuit de traitement (106) est en outre configuré pour recevoir des données de pression provenant des capteurs de pression (404).

8. Système (100) selon la revendication 7, dans lequel la détermination des transducteurs ultrasoniques (402) qui sont en contact avec l'anatomie (406) du patient est en outre basée sur les données de pression.

9. Système (100) selon la revendication 1, dans lequel le modèle d'os est basé sur au moins l'un d'un tomodensitogramme ou d'une IRM.

10. Système (100) selon la revendication 1, dans lequel le modèle d'os est un modèle obtenu à l'aide d'un système sans image.

11. Support lisible par ordinateur non transitoire sur lequel sont stockées des instructions pour exécution par un circuit de traitement (106), les instructions comprenant :
des instructions pour recevoir des données ultrasoniques de l'anatomie d'un patient (304 ; 406) en provenance d'une sonde d'alignement qui comporte un réseau d'une pluralité de transducteurs ultrasoniques (110 ; 204 ; 302 ; 402) couplés à un bout arrondi d'une sonde, dans lequel les transducteurs ultrasoniques (110 ; 204 ; 302 ; 402) sont configurés pour fournir des données ultrasoniques de l'anatomie du patient (304 ; 406) ;
des instructions pour déterminer lesquels des transducteurs ultrasoniques (110 ; 204 ; 302 ; 402) sont en contact avec l'anatomie du patient (304 ; 406) ;
des instructions pour aligner l'anatomie du patient (304 ; 406) avec un modèle d'os à l'aide de points d'alignement ; **caractérisé en ce que** les instructions comprennent en outre :
des instructions pour sélectionner un sous-ensemble de la pluralité de transducteurs ultrasoniques (110 ; 204 ; 302 ; 402) d'après la détermination des transducteurs ultrasoniques (110 ; 204 ; 302 ; 402) qui sont en contact avec l'anatomie du patient (304 ; 406), les transducteurs ultrasoniques du sous-ensemble étant les transducteurs ultrasoniques qui sont en contact avec l'anatomie du patient ;
des instructions pour générer les points d'alignement d'après les données ultrasoniques du sous-ensemble sélectionné de transducteurs ultrasoniques (110 ; 204 ; 302 ; 402).

12. Support lisible par ordinateur non transitoire selon la revendication 11, comprenant en outre des instructions pour recevoir des données de pression en provenance de capteurs de pression (404), dans lequel la sonde d'alignement (108 ; 200) comporte en outre une pluralité de capteurs de pression (404) couplés au bout arrondi (400) de la sonde (108 ; 200), dans lequel les capteurs de pression (404) sont configurés pour fournir des données de pression, et dans lequel la détermination des transducteurs ultrasoniques (402) qui sont en contact avec l'anatomie du patient (406) est en outre basée sur les données de pression.
